**Europäisches Patentamt**

(19) **European Patent Office** (11) Publication number: **0 107 885**

**Office européen des brevets** **B1**

(12) **EUROPEAN PATENT SPECIFICATION**

(45) Date of publication of patent specification: **29.07.87** (51) Int. Cl.⁴: **A 61 K 7/06, A 61 K 7/48**

(21) Application number: **83201530.9**

(22) Date of filing: **25.10.83**

(54) Cosmetic preparations promoting the trophism of the skin and of the related hair follicles.

(30) Priority: **29.10.82 IT 2399482**
**13.07.83 IT 2204783**

(43) Date of publication of application:
**09.05.84 Bulletin 84/19**

(45) Publication of the grant of the patent:
**29.07.87 Bulletin 87/31**

(84) Designated Contracting States:
**AT BE DE GB NL SE**

(56) References cited:
**DE-A-2 317 138**
**FR-A-1 303 571**
**FR-A-1 568 721**
**FR-A-2 160 285**
**FR-A-2 472 385**
**DR. OTTO-ALBRECHT NEUMÜLLER: "Römpps Chemie-Lexikon", vol. 2, 7th edition, 1973, Franchk'sche Verlagshandlung, W. Keller & Co., Stuttgart, DE.**

**The file contains technical information submitted after the application was filed and not included in this specification**

(73) Proprietor: **Crinos Industria Farmacobiologica S.p.A.**
**Piazza XX Settembre, n. 2**
**I-22079 Villa Guardia Como (IT)**

(72) Inventor: **Gazzani, Giovanni**
**Via A.Volta 28**
**Appiano Gentile (Como) (IT)**

(74) Representative: **Dragotti, Gianfranco**
**SAIC BREVETTI s.a.s. Viale Bianca Maria, 15**
**I-20124 Milano (IT)**

(56) References cited:
**SEIFEN-ÖLE-FETTE-WACHSE, vol. 107, no. 18, November 1981, page 570, Augsburg, DE.**
**KC BIOLOGICAL INC CATALOGUE, "Products for Tissue Culture", 1981**

**0 107 885**

### Description

The present invention relates to a cosmetic preparation for promoting the trophism of the cutis and of the hair follicles. More particularly the present invention relates to a cosmetic preparation to be used as a revitalizing agent for the skin and as active factor for hair growth.

When the skin is pale, wrinkly, of low elasticity and dehydrated and when it shows clear symptoms of suffering and of premature ageing, it is most likely that its germinative layer is more or less seriously atrophied.

It is in fact known that the germinative layer of the skin consists of continuously reproducing and growing cells and that it requires continuous feeding of nutrient substances for it to be maintained in a vital and efficient condition.

It is known as well that such feeding is prevailingly, if not exclusively, entrusted to the blood circulation.

When the blood circulation towards and within the germinative layer is hindered, or the feeding of nutrient substances is reduced, said layer becomes more and more atrophied and the skin becomes wrinkly, pale and "old".

In the particular case of the hair, for the matrix and the papilla, which are involved by an intense metabolism, it is even more necessary to make available in a relevant amount the nutrient substances required for their trophism: as it is known, a lack of said substances would firstly, cause the weakening and then the atrophy of the hair follicle, with attendant lack of the capacity of forming new hair.

The cosmetic preparations for more or less specific topical treatment of the skin which have been widely used up to date in order to attenuate the aforesaid drawbacks, are in most cases mere palliatives, the efficacy of which has well known limits, essentially due to the fact that it is not possible through them to provide the skin germinative layer the or those few nutrient substances which said preparations may comprise in their composition.

In the literature too, several proposals are described in detail for the solution of the aforesaid problem.

a) In the German published application No. 2,317,138 a preparation against hair fall is described, which consists essentially of a serum extracted from snakes and animals, particularly from snakes, which is used either as such or suitably concentrated and processed.

The specification also contemplates the addition to the serum of simple molecules, such as aminoacids, vitamins, hormones, etc.

b) In French Patent No. 2,472,385 the use is taught, in the cosmetic field, of the product from the thermal cracking of serums, tissue extracts and the like, the cracking being carried out until peptides of molecular weight of between 5000 and 50,000 are obtained. It is in fact known that these peptides have the property of influencing cell growth.

c) in French Patent No. 2,160,285 the use of a lyophilized product is foreseen in the cosmetic field. This product is obtained from several serums, amniotic liquid or placenta.

d) French patent No. 1,303,571 teaches the use, in the cosmetic field, of preparations containing one or more biocatalyst complexes, mainly of proteic nature, formed by combining biochemical or chemical compounds showing a certain nutrient action toward the skin, with biomedical products suitable for accelerating the cutaneous cellular metabolism, such as hormones, vitamins, etc.

The common feature of all these products and preparations is their biological and extractive origin, whereby their production cannot be adequately standardized, with the evident consequences, especially as regards the consistency of the effects.

Moreover, the fact that not only the presence or absence, but also the amount of the single components of the subject mixtures seem to be important cannot be neglected.

For the culture *in vitro* of epithelial cells the use of a synthetic nutrient medium comprising a mixture of simple and quantitatively defined molecules is known.

It is also known that the synthetic medium, in order to obtain a longer action on the cultures, must be supplemented with a certain amount of a natural medium.

KD Biological Inc. Catalogue, "Products for Tissue culture", 1981, page. 28, discloses a composition comprising a nutrient medium and foetal bovine serum for the *in vitro* culture of isolated human epithelial cells.

The problem not yet solved is that of providing a cosmetic preparation which not only comprises all the nutrient substances which are essential in order to maintain in the vital and efficient condition of the germinative layer of the skin and the piliferous papilla, but also ensures a substantial contribution, balanced and complete, of these substances thereto.

The aforesaid problem is solved according to the present invention by means of a cosmetic preparation for the topical treatment of the skin in order to promote its cellular trophism and of the related piliferous follicles, characterized by comprising, as a percent by weight of said preparation, 0.1 to 1% of a nutrient medium for *in vitro* culture of isolated human epithelial cells and 2 to 10% of foetal bovine serum, based on the amount of said nutrient medium, said nutrient medium being devoid of mineral salts.

If account is taken of the conventional mode of application of a cosmetic preparation and of the well known impermeability of the skin towards almost all the aforesaid nutrient substances (since the factor limiting the cutaneous absorption is, as it is well known, the horny layer of the skin), said nutrient

2

substances should be preferably present in their simplest form with the lowest molecular weight: for instance aminoacids instead of proteins, nucleic bases instead of nucleic acids, pentoses and hexoses instead of polysaccharides, etc.

Moreover, to promote the permeation of said nutrient substances through the skin, the cosmetic preparation of the present invention comprises dermophilic vehicles, such as for instance ethyl alcohol, polyalcohols or glycols and emulsifiers, of the type conventionally used for such a purpose.

It is known that lotions for topical use based on mixtures obtained from the extraction of connective tissues of animals organs (such as for example Trichosaccharide) mainly comprising sulphomucopolysaccharides (or glycosaminoglycosanes), can be advantageously used in order to bring to normality the functions of the piliferous papillae and of the related germinative cells.

The action of these substances involves the small blood vessels which irrigate the pilosebaceous follicle, restoring the normal conditions of local circulation. The concentration of this active substance in the compositions may vary from 0.1 to 10%, preferably 0.3 to 0.6%, based on 100 parts by weight of the composition.

It has now been surprisingly found that the combination of these substances with the previously mentioned active compounds and which are the subject of the present invention, gives rise to a composition which does not only act simultaneously on both causes leading to the hair fall (local circulatory lack and insufficient contribution of nutrient elements), but also shows an unexpectedly greater activity with respect to that foreseeable from the sole sum of the single components.

According to the invention it has been in fact found that the mixtures obtained by extraction and the nutrient compositions for medias of cellular cultures show, within some ratios, a relevant synergistic action with an improvement in the efficacy of both active substances, the combination of which acts moreover, as mentioned above, on all the causes of hair fall.

It is thus a further object of the present invention to provide a synergistic composition for the stimulation of the trichogenesis which is characterized by comprising a first active component consisting of mixtures obtained by extraction of the connective tissus of animal organs and a second active component consisting of a nutrient medium for the *in vitro* culture of human epithelial cells.

It is known that a nutrient medium for the *in vitro* culture of isolated human cells contains all the nutrient elements necessary for the life of the cells and for their survival and reproduction.

The topical application of a cosmetic preparation according to the invention, which is an *in vivo* application generally carried out in the skin areas wherein the blood irrigation and thus the feeding of nutrient substances is most hindered and slow, has been proved to cause a substantial improvement in the local trophic conditions and a consequent normalization of the vitality and reproducing activity of the cells.

It has moreover been found that the mineral salts, normally present in the medium for the *in vitro* culture of isolated human cells, must be absent from the nutrient medium incorporated in the cosmetic preparation according to the present invention. As a matter of fact, whilst the mineral salts, in the strict sense, are not nutrient substances, on the other hand they are already present in the cellular liquids. Moreover, in order to take into account the unavoidable dispersion of nutrient substances at the time of the application of a cosmetic preparation according to the invention, the concentration of the nutrient substances in the medium incorporated in said preparation must be 3 to 5 times greater than the concentration of said substances in the like medium used for the *in vitro* culture of isolated human epithelial cells.

Further features of the present invention shall appear more clearly from the following description in which a composition of a nutrient medium is illustrated for the use in a preparation according to the invention and wherein some examples are given of cosmetic formulations based on the aforesaid product.

**0 107 885**

Example of composition of a powder nutrient medium to be incorporated in a cosmetic
preparation according to the invention (percentage formula)

| | | | | | |
|---|---|---|---|---|---|
| L-Alanine | g | 3.1997 | Choline hydrochloride | g | 0.0639 |
| L-Arginine | g | 8.9595 | Folic acid | g | 0.0012 |
| L-Aspartic acid | g | 3.8397 | i-Inositol | g | 0.0063 |
| L-Cystine disodium salt | g | 3.0282 | Menaphtone Na-bisulphite | g | 0.0024 |
| L-Cysteine HCl | g | 0.0126 | Nicotinic acid | g | 0.0031 |
| L-Glutamic acid | g | 8.5524 | Nicotinamide | g | 0.0031 |
| L-Glutamine | g | 12.7991 | p-Aminobenzoic acid | g | 0.0063 |
| Glutathione | g | 0.0063 | Pyridoxal HCl | g | 0.0031 |
| Glycine | g | 6.4086 | Pyridoxine HCl | g | 0.0031 |
| L-Histidine HCl.H$_2$O | g | 2.8004 | Riboflavine | g | 0.0012 |
| L-Hydroxyproline | g | 1.2799 | Thiamine HCl | g | 0.0012 |
| L-Isoleucine | g | 2.5598 | DL-Tocopherol Na phosphate | g | 0.0012 |
| L-Leucine | g | 7.6795 | Vitamin A acetate | g | 0.0146 |
| L-Lysine HCl | g | 8.9594 | Adenine sulfate | g | 1.2799 |
| L-Methionine | g | 1.9198 | 5-AMP | g | 0.0256 |
| L-Phenylalanine | g | 3.1997 | ATP disodium salt | g | 1.2799 |
| L-Proline | g | 5.1196 | S-Deoxyribose | g | 0.0639 |
| L-Serine | g | 3.1997 | Hypoxanthine | g | 0.0384 |
| L-Tryptophan | g | 1.2799 | Ribose | g | 0.0639 |
| L-Tyrosine | g | 5.1196 | Thymine | g | 0.0384 |
| L-Valine | g | 3.1997 | Uracil | g | 0.0384 |
| L-Ascorbic acid | g | 0.0063 | Xanthine | g | 0.0384 |
| Biotin | g | 0.0012 | | | |
| Calciferol | g | 0.0127 | | | |
| D-calcium pantothenate | g | 0.0012 | | | |

This composition is prepared by operating in an environnment with a relative humidity of less than 40%; all the components are weighed in the order shown and the resulting mixture is charged into a ball mill where it is finely ground. The resulting powder is stored protected from moisture.
Examples of formulations for anti-wrinkle, hydrating and revitalizing products for the skin.

### Example 1
Cream

| | | |
|---|---|---|
| Medium of the invention | g | 0.4 |
| foetal bovine serum | g | 2.5 |
| polyglycol stearate | g | 5.0 |
| stearin | g | 6.5 |
| lanolin oil | g | 6 |
| squalane | g | 2 |
| spermaceti | g | 8 |
| preservatives and perfume | q.b. | |
| H$_2$O | to g | 100 |

### Example 2
Milk

| | | |
|---|---|---|
| Medium of invention | g | 0.3 |
| foetal bovine serum | g | 2 |
| polyoxyethyleneglycol palmitostearate | g | 5 |
| lanolin oil | g | 5 |
| glyceryl monostearate | g | 1 |
| propylene glycol | g | 4 |
| preservatives and perfumes | q.a. | |
| H$_2$O | to g | 100 |

4

### Example 3

Lotion

| Concentrated medium | | g 0.2 |
|---|---|---|
| foetal bovine serum | | g 2.5 |
| glucose | | g 0.1 |
| alcohol 95° | | ml 18 |
| Hamamelis extract | | g 1 |
| ethoxylated lanolin | | g 2 |
| glycerin | | g 5 |
| preservatives and perfumes | | q.b. |
| H$_2$O | to | ml 100 |

### Examples of lotions for the hair growth
### Example 4

| Concentrated medium | | g 0.5 |
|---|---|---|
| foetal bovine serum | | g 8 |
| glucose | | g 0.1 |
| alcohol 95° | | ml 3.5 |
| preservatives and perfumes | | q.b. |
| H$_2$O | to | ml 100 |

### Example 5

| Concentrated medium | | g 0.3 |
|---|---|---|
| foetal bovine serum | | g 5 |
| alcohol 95° | | ml 20 |
| propylene glycol | | g 3 |
| preservatives and perfume | | q.b. |
| H$_2$O | to | ml 100 |

### Example 6

| Concentrated medium | | g 0.2 |
|---|---|---|
| foetal bovine serum | | g 2 |
| Tween 80® | | g 0.01 |
| nettle extract | | g 3 |
| capsicum extract | | g 1 |
| preservatives and perfume | | q.b. |
| H$_2$O | to | ml 100 |

As previously mentioned, within the scope of the invention it has also been found that the above described nutrient compositions and the mixtures obtain by extraction of connective tissues of animal organs show, within some ranges, a relevant synergistic action, with the attendant improvement in the efficacy of both active substances. As already indicated, amongst the active agents for the stimulation of the local blood circulation, there are preferred for the present invention the mixtures extracted from connective tissues of animal organs, known as "Trichosaccaride", in the form of lotions. Of course in the present invention use can be made of other already known active components, as vegetal extracts, such as nettle, Hamamelis, birch extracts or other components capable of stimulating the scalp as vasodilating agents, such as capsaicin, esters of the nicotinic acid (for instance, methyl and ethyl nicotinate).

Hereinafter some examples of formulations of the active components of the preparation of the invention and of their mixtures are reported.

### Example 7
Example of composition of the lotion based on the extractive mixture (composition A).

| 95% ethyl alcohol | | ml 15 |
|---|---|---|
| propylene glycol | | g 1.5 |
| preservative and perfume | | g 0.8 |
| extracted mixture (Trichosaccharide) | | g 1 |
| water | to | ml 100 |

# 0 107 885

## Example 8
### Examples of composition of medium and foetal bovine serum and vehicle according to the invention (composition B)

| | |
|---|---|
| Medium+foetal bovine serum | g 62.5 |
| inositol | g 12.5 |
| mannitol | g 25 |

## Example 9
### Examples of synergistic lotions according to the present invention (composition C).

| | | | | |
|---|---|---|---|---|
| Component B (mg) | 100 | 400 | 700 | 1000 |
| Component A (g or ml) | 100 | 100 | 100 | 100 |
| Concentration of B | 0.1% | 0.4% | 0.7% | 1% |

The composition C of the invention was compared with the preparation used in the prior art, namely lotions based on extractive mixtures (composition A).

For the experiments five tawny rabbits (Burgundy) were used, the animals being normally fed, weighing 2.8 kg and being previously shaven on all the dorsal area.

In order to more rapidly show the results of the experiments it was preferred to subcutaneously inject the preparations, instead of applying them topically since the stimulation of the new growth is not revealed in the animal by topical application.

The day after the shaving, 0.1 ml of the following solutions, prepared immediately before use, were injected:

1) extracted mixture (composition A) in 1% w/v aqueous solution;
2) 1% extracted mixture and 0.5% of composition B (weight by volume) in aqueous solution.

The two injections were carried out in the back, at two different heights and in a double test for each solution. The injections were repeated for two periods of five days in succession, with an interval of two rest days.

The tests gave the following results.

The solution (1) caused fresh growth of the coat or hair around the injection area.

The fresh growth was clearly visible at about 15 days from the beginning of the injection. The solution (2) caused a more rapid fresh growth, which moreover involved a more extended area with respect to that obtained, for each animal, with the sole solution (1).

As already mentioned, the present invention relates also to an anti-wrinkle, hydrating and revitalizing action for the skin. In the following Examples 10 to 13 the preparation of a cream, a milk and a lotion is illustrated.

## Example 10

A planetary mixer of stainless steel is charged with 90% of the water necessary for the preparation of the cream and then the temperature is raised to 70 to 80°C.

Parallely a separate melter is charged with polyglycol stearate, stearin, lanolin oil, squalane and preservatives, bringing the temperature to a value of between 70 and 80°C and starting the mixing action as soon as possible.

When the temperatures of the aqueous phase and of the fatty phase are in equilibrium, the fatty phase is poured into the water. The planetary mixing is started and the mixture is slowly cooled.

When the mass has reached 35°C, there are added the concentrated medium, previously dispersed in the 10% of the water, the foetal bovine serum and the perfume. Then the cooling is completed by keeping the mass under stirring.

## Example 11

A planetary mixer of stainless steel is charged with 90% of the water needed for the preparation of the milk and with the propylene glycol and the temperature is brought to a value of between 70°C and 80°C.

Parallelly, a separate mixer is charged with polyglycol stearate, lanolin oil, glyceryl monostearate and the preservatives, bringing the temperature to a value of between 70°C and 80°C and starting the mixing as soon as possible.

When the temperatures of the aqueous phase and of the fatty phase are in equilibrium, the fatty phase is poured into the water. The planetary mixing is started and the mass is slowly cooled.

When the mass has reached 35°C, there are added the concentrated medium, as a predispersion in 10% of the water, the foetal bovine serum and the perfume. The cooling is then completed by keeping the mass always under stirring.

6

## 0 107 885

Example 12

A vessel of stainless steel, fitted with a stirrer is charged in the following order with 90% of the needed water, then with the concentrated medium, the foetal bovine serum, the glucose, the Hamamelis extract, the glycerin, the preservatives and the perfume.

The mixing is started and then the ethoxylate lanoline is added, in a form predissolved in the remaining 10% of hot water. The mass is stirred for 15 minutes and then alcohol added and stirred again.

The pH is brought to 6.5 with dilute sodium hydroxide or HCl, then the mass is filtered through a filter of the type Seitz US and then bottled.

Example 13

A stainless steel vessel, fitted with a stirrer, is charged with the necessary water and then with all the components according to Example 7, whereas the solid mixture is separately prepared according to Example 8. The mass is stirred for about 20 minutes, and then controlled to verify whether the pH is 6.5; if necessary the pH is adjusted with dilute sodium hydroxide or HCl. After controlling the final volume the mass is stirred again for five minutes and the solution is allowed to stand overnight, and then filtered through a clarifying filter of the type Seitz US.

After filtration, the packaging is carried out, comprising the bottling of the solution and the simultaneous dosing of the solid mixture in the reservoir-plug.

**Claims**

1. Cosmetic preparation for promoting the trophism of the skin and of the related piliferous follicles, characterized by comprising, as a percent weight of said preparation, 0.1 to 1% of a nutrient medium for *in vitro* culture of isolated human epithelial cells and 2 to 10% of foetal bovine serum based on the amount of said nutrient medium, said nutrient medium being devoid of mineral salts.

2. Cosmetic preparation according to claim 1, characterized in that said nutrient medium has the following composition as a percentage formulation:

| | | | | | |
|---|---|---|---|---|---|
| L-Alanine | g | 3.1997 | Choline hydrochloride | g | 0.0639 |
| L-Arginine | g | 8.9595 | Folic acid | g | 0.0012 |
| L-Aspartic acid | g | 3.8397 | i-Inositol | g | 0.0063 |
| L-Cystine disodium salt | g | 3.0282 | Menaphtone Na-bisulphite | g | 0.0024 |
| L-Cysteine HCL | g | 0.0126 | Nicotinic acid | g | 0.0031 |
| L-Glutamic acid | g | 8.5524 | Nicotinamide | g | 0.0031 |
| L-Glutamine | g | 12.7991 | p-Aminobenzoic acid | g | 0.0063 |
| Glutathione | g | 0.0063 | Pyridoxal HCl | g | 0.0031 |
| Glycine | g | 6.4086 | Pyridoxine HCl | g | 0.0031 |
| L-Histidine $HCl.H_2O$ | g | 2.8004 | Riboflavine | g | 0.0012 |
| L-Hydroxyproline | g | 1.2799 | Thiamine HCl | g | 0.0012 |
| L-Isoleucine | g | 2.5598 | DL-Tocopherol Na phosphate | g | 0.0012 |
| L-Leucine | g | 7.6795 | Vitamin A acetate | g | 0.0146 |
| L-Lysine HCl | g | 8.9594 | Adenine sulfate | g | 1.2799 |
| L-Methionine | g | 1.9198 | 5-AMP | g | 0.0256 |
| L-Phenylalanine | g | 3.1997 | ATP disodium salt | g | 1.2799 |
| L-Proline | g | 5.1196 | S-deoxyribose | g | 0.0639 |
| L-Serine | g | 3.1997 | Hypoxanthine | g | 0.0384 |
| L-Tryptophan | g | 1.2799 | Ribose | g | 0.0639 |
| L-Tyrosine | g | 5.1196 | Thymine | g | 0.0384 |
| L-Valine | g | 3.1997 | Uracil | g | 0.0384 |
| L-Ascorbic acid | g | 0.0063 | Xanthine | g | 0.0384 |
| Biotin | g | 0.0012 | | | |
| Calciferol | g | 0.0127 | | | |
| D-calcium pantothenate | g | 0.0012 | | | |

3. Cosmetic preparation according to claim 2, characterized by furthermore comprising a dermophilic vehicle selected from ethyl alcohol, polyalcohols or glycols and emulsifiers.

4. Cosmetic preparation according to claim 1, characterized by further comprising an active ingredient consisting of mixtures obtained by extraction of connective tissues of animal organs.

5. Cosmetic preparation according to claim 4, characterized in that the concentration in percent by weight of said second active ingredient, based on 100 parts by weight of the composition, is between 0.1 and 10%.

6. Cosmetic preparation according to claim 5, characterized in that said concentration in percent by weight is between 0.3 and 0.6%.

7. Cosmetic preparation according to claim 4, characterized in that said active ingredient is Trichosaccharide.

7

8. Cosmetic preparation according to claim 1, characterized by being in a form suitable for use as a cream, hydrating-revitalizing milk or hair lotion.

9. Cosmetic preparation according to claim 4, characterized by being in a form suitable for use as a cream, hydrating-revitalizing milk or hair lotion.

## Patentansprüche

1. Kosmetische Zubereitung zur Förderung der Ernährung der Haut und der damit verwandten behaarten Follikel, dadurch gekennzeichnet, daß sie, ausgedrückt als Gew.-% der genannten Zubereitung, 0,1 bis 1% eines Nährmediums für die in-vitro-Kultur von isolierten Humanepithelzellen und 2 bis 10% Rinderfötalserum, bezogen auf die Menge des genannten Nährmediums, enthält, wobei das genannte Nährmedium von Mineralsalzen frei ist.

2. Kosmetische Zubereitung nach Anspruch 1, dadurch gekennzeichnet, daß das genannte Nährmedium die folgende Zusammensetzung als prozentuale Formulierung aufweist:

| | | | |
|---|---|---|---|
| L-Alanin | 3,1997 g | Cholinhydrochlorid | 0,0639 g |
| L-Arginin | 8,9595 g | Folsäure | 0,0012 g |
| L-Asparaginsäure | 3,8397 g | i-Inosit | 0,0063 g |
| L-Cystindinatrium-salz | 3,0282 g | Menaphton-Na-bisulfit | 0,0024 g |
| L-Cystein HCl | 0,0126 g | Nikotinsäure | 0,0031 g |
| L-Glutaminsäure | 8,5524 g | Nikotinamid | 0,0031 g |
| L-Glutamin | 12,7991 g | p-Aminobenzoesäure | 0,0063 g |
| Glutathion | 0,0063 g | Pyridoxal HCl | 0,0031 g |
| Glycin | 6,4086 g | Pyridoxin HCl | 0,0031 g |
| L-Histidin HCl.H$_2$O | 2,8004 g | Riboflavin | 0,0012 g |
| L-Hydroxyprolin | 1,2799 g | Thiamin HCl | 0,0012 g |
| L-Isoleucin | 2,5598 g | DL-Tocopherol-Na-Phosphat | 0,0012 g |
| L-Leucin | 7,6795 g | Vitamin-A-Acetat | 0,0146 g |
| L-Lysin HCl | 8,9594 g | Adeninsulfat | 1,2799 g |
| L-Methionin | 1,9198 g | 5-AMP | 0,0256 g |
| L-Phenylalanin | 3,1997 g | ATP-Dinatrium-salz | 1,2799 g |
| L-Prolin | 5,1196 g | S-Deoxyribose | 0,0639 g |
| L-Serin | 3,1997 g | Hypoxanthin | 0,0384 g |
| L-Tryptophan | 1,2799 g | Ribose | 0,0639 g |
| L-Tyrosin | 5,1196 g | Thymin | 0,0384 g |
| L-Valin | 3,1997 g | Uracil | 0,0384 g |
| L-Ascorbinsäure | 0,0063 g | Xanthin | 0,0384 g |
| Biotin | 0,0012 g | | |
| Calciferol | 0,0127 g | | |
| D-Calciumpantothenat | 0,0012 g | | |

3. Kosmetische Zubereitung nach Anspruch 2, dadurch gekennzeichnet, daß sie weiterhin einen dermophilen Träger, ausgewählt aus Ethylalkohol, Polyalkoholen oder Glykolen und Emulgatoren, enthält.

4. Kosmetische Zubereitung nach Anspruch 1, dadurch gekennzeichnet, daß sie weiterhin einen Wirkstoff bestehend aus Gemischen, erhalten durch Extraktion von Verbindungsgeweben von tierischen Organen, enthält.

5. Kosmetische Zubereitung nach Anspruch 4, dadurch gekennzeichnet, daß die in Gew.-% ausgedrückte Konzentration des genannten zweiten Wirkstoffs, bezogen auf 100 Gew.-Teile der Zubereitung, zwischen 0,1 und 10% liegt.

6. Kosmetische Zubereitung nach Anspruch 5, dadurch gekennzeichnet, daß die genannte, auf das Gewicht bezogene Konzentration zwischen 0,3 und 0,6% liegt.

7. Kosmetische Zubereitung nach Anspruch 4, dadurch gekennzeichnet, daß der genannte Wirkstoff Trichosaccharid ist.

8. Kosmetische Zubereitung nach Anspruch 1, dadurch gekennzeichnet, daß sie in einer Form vorliegt, die zur Verwendung als Creme, hydratisierende revitalisierende Milch oder Haarlotion geeignet ist.

9. Kosmetische Zubereitung nach Anspruch 4, dadurch gekennzeichnet, daß sie in einer Form vorliegt, die zur Verwendung als Creme, hydratisierende revitalisierende Milch oder Haarlotion geeignet ist.

## Revendications

1. Préparation cosmétique pour favoriser le trohisme de la peau et des follicules pilifères apparentés, caractérisée en ce qu'elle comprend, en pourcentage en poids de la préparation, 0,1 à 1% d'un milieu nutritif pour la culture in vitro de cellules épithéliales humaines isolées et 2 à 10% de sérum bovin foetal par rapport à la quantité du milieu nutritif, ce milieu nutritif étant dépourvu de sels minéraux.

2. Préparation cosmétique suivant la revendication 1, caractérisée en ce que le milieu nutritif répond à la composition suivante sous la forme d'une formulation en pourcentage:

| | | | |
|---|---|---|---|
| L-Alanine | 3,1997 g | Chlorhydrate de choline | 0,0639 g |
| L-Arginine | 8,9595 g | Acide folique | 0,0012 g |
| Acide L-aspartique | 3,8397 g | i-Inositol | 0,0063 g |
| Sel disodique de L-cystine | 3,0282 g | Na-bisulfite de ménaphtone | 0,0024 g |
| L-Cystéine HCl | 0,0126 g | Acide nicotinique | 0,0031 g |
| Acide L-glutamique | 8,5524 g | Nicotinamide | 0,0031 g |
| L-Glutamine | 12,7991 g | Acide p-aminobenzoïque | 0,0063 g |
| Glutathione | 0,0063 g | Pyridoxal HCl | 0,0031 g |
| Glycine | 6,4086 g | Pyridoxine HCl | 0,0031 g |
| L-Histidine HCl.H$_2$O | 2,8004 g | Riboflavine | 0,0012 g |
| L-Hydroxyproline | 1,2799 g | Thiamine HCl | 0,0012 g |
| L-Isoleucine | 2,5598 g | Na-phosphate de DL-tocophérol | 0,0012 g |
| L-Leucine | 7,6795 g | Acétate de vitamine A | 0,0146 g |
| L-Lysine HCl | 8,9594 g | Sulfate d'adénine | 1,2799 g |
| L-Méthionine | 1,9198 g | 5-AMP | 0,0256 g |
| L-Phénylalanine | 3,1997 g | Sel disodique d'ATP | 1,2799 g |
| L-Proline | 5,1196 g | S-Déoxyribose | 0,0639 g |
| L-Sérine | 3,1997 g | Hypoxanthine | 0,0384 g |
| L-Tryptophane | 1,2799 g | Ribose | 0,0639 g |
| L-Tyrosine | 5,1196 g | Thymine | 0,0384 g |
| L-Valine | 3,1997 g | Uracile | 0,0384 g |
| Acide L-ascorbique | 0,0063 g | Xanthine | 0,0384 g |
| Biotine | 0,0012 g | | |
| Calciférol | 0,0127 g | | |
| D-Pantothénate de calcium | 0,0012 g | | |

3. Préparation cosmétique suivant la revendication 2, caractérisée en ce qu'elle comprend en outre un véhicule dermophile choisi dans le groupe comprenant l'alcool éthylique, les polyalcools ou les glycols et les émulsifiants.

4. Préparation cosmétique suivant la revendication 1, caractérisée en ce qu'elle comprend en outre un ingrédient actif se composant de mélanges obtenus par extraction de tissus conjonctifs d'organes d'animal.

5. Préparation cosmétique suivant la revendication 4, caractérisée en ce que la concentration en % en poids du second ingrédient actif, par rapport à 100 parties en poids de la composition, se situe entre 0,1 et 10%.

6. Préparation cosmétique suivant la revendication 5, caractérisée en ce que la concentration en % en poids se situe entre 0,3 et 0,6%.

7. Préparation cosmétique suivant la revendication 4, caractérisée en ce que l'ingrédient actif est le Trichosaccharide.

8. Préparation cosmétique suivant la revendication 1, caractérisée en ce qu'elle est sous une forme appropriée pour être utilisée comme crème, lait hydratant-revitalisant ou lotion capillaire.

9. Préparation cosmétique suivant la revendication 4, caractérisée en ce qu'elle est sous une forme appropriée pour être utilisée comme crème, lait hydratant-revitalisant ou lotion capillaire.